# EUROPEAN PATENT SPECIFICATION

(11) **EP 4 162 975 B1**
(45) Date of publication and mention of the grant of the patent: **30.07.2025**
(21) Application number: 22208459.2
(22) Date of filing: 23.10.2020
(51) Int. Cl.: A61N 1/362

(54) **USER-ADJUSTABLE EXTERNAL CARDIAC PACEMAKER**
EXTERNER HERZSCHRITTMACHER MIT EINSTELLBARER BENUTZERFUNKTION
STIMULATEUR CARDIAQUE EXTERNE RÉGLABLE PAR L'UTILISATEUR

(43) Date of publication of application: 12.04.2023
(62) Divisional of application: 20203668.7
(73) Proprietor: Osypka Medical GmbH, 12489 Berlin (DE)
(72) Inventor: OSYPKA, Markus, 79639 Grenzach-Wyhlen (DE); STEINKE, Clemens, 12555 Berlin (DE); ZELMER, Dirk, 12557 Berlin (DE); SCHNABEL, Rainer, 15741 Bestensee (DE); ROSE, Marcus, 10179 Berlin (DE)
(74) Representative: Röthinger, Rainer

(56) References cited:
- WO-A2-2007/075732
- US-A- 5 623 936
- US-A- 5 792 192
- US-A1- 2015 157 231

## Description

### Technical Field

The present disclosure generally relates to cardiac pacemakers. In more detail, a technique involving a user-adjustable external cardiac pacemaker is presented. The technique may be implemented in the form of a method, a training system and a user-adjustable external cardiac pacemaker.

### Background

Cardiac pacing is a well-established medical therapy for which implantable, or internal, cardiac pacemakers as well as external, or temporary, cardiac pacemakers have been developed. Those two cardiac pacemaker types are collectively referred to as pacemakers hereinafter.

Temporary cardiac pacing using external cardiac pacemakers is employed in, but not limited to, the emergency room, intensive care unit and operating room. Typical applications of external cardiac pacemakers are transvenous pacing, temporary pacing via heart wires and external transthoracic pacing.

Proper control of pacemaker operation is of utmost importance for the patient's health. For this reason, pacemakers typically offer the possibility to adjust several of its operational parameters by a trained medical professional to control pacemaker operation, including the generation of electric stimuli. An external pacemaker comprises a user interface for parameter adjustment (e.g., a mechanical interface with buttons or switches). In the case of an implantable pacemaker, parameter adjustments are performed using a software tool and communicated to the pacemaker via a wireless transmission link.

The basic function of a pacemaker is to generate electric stimuli which trigger myocardial depolarization. Myocardial depolarization is also referred to as "capture". Failure to capture when a pacemaker is deployed may be due to insufficient stimulation amplitude of an electric stimulus (i.e., an overly low pacing voltage) or insufficient pulse width of the stimulus, failure of the stimulus to excite the tissue surrounding the lead system (also referred to as "exit block"), or technical obstacles such as problems with the pacing lead or lead extension. It is good hospital practice to have a trained medical professional manually determine the capture threshold upon deployment of the pacemaker and set the stimulation amplitude at a level of about 2-3 times of the capture threshold.

State-of-the-art pacemakers provide demand pacing modes which stimulate the heart only if the pacemaker does not recognize intrinsic activity within a specific expectation window. Proper recognition of intrinsic activity ("sensing") requires establishment of a sensing threshold, which often is a manual process according to good hospital practice. Commonly, the sensing threshold is set between about one-half (1/2) or one-third (1/3) of the amplitude of a so-called P wave in an atrial chamber and a so-called R wave in a ventricular chamber.

In addition to setting, or programming, an appropriate stimulation amplitude and a sensing threshold, selection of the appropriate stimulation therapy, or pacing mode, is of importance for proper heart stimulation. Good hospital practice requires that a medical professional is trained on and familiar with recognizing A-V blocks and atrial fibrillation and to set the pacing parameters accordingly.

While well-trained and experienced medical staff generally has no difficulty to execute such parameter adjustments even during demanding emergency situations, it is the occasional user of in particular a temporary pacemaker who may struggle to deploy the pacemaker when a patient is in dire need of a pacing therapy. An annual training or in-service has been found to be insufficient to maintain the skills in pacing therapy for the occasional user.

For this reason, there is a need for a technique that permits controlling operation of a user-adjustable cardiac pacemaker, or a software model thereof, in an efficient training mode. Also, there is a need for a pacemaker, in particular an external pace-maker, that can efficiently be employed for user training.

WO 2007/075732 A2 relates to a cardiac pacemaker with multifunctional pacing modes that can be used for temporary intra- or extra-single/dual chamber cardiac pacing. An LCD screen is integrated in this portable device to provide a real-time electrocardiograph (ECG) monitoring. This device displays intra-chamber ECG that could guide a physician to catheterize the heart without X-ray machine/fluoroscopy or other guiding equipment. It also can be used as an independent long-term surface ECG monitor. The device is equipped with a clear-cut circuit design, a three level switching system for managing electrophysiological stimulation, an interface for person/machine dialog, and cardiac signal recording.

US 2015/0157231 A1 discloses systems, methods, and interfaces for assisting a user in noninvasive evaluation of patients for cardiac therapy and noninvasive evaluation of cardiac therapy being delivered. The systems, methods, and interfaces may provide graphical representations of cardiac electrical activation times about one or more portions of human anatomy and one or mare cardiac health metrics.

US 5,792,192 relates to a rate responsive pacemaker in which an AMS (automatic mode switching) feature is selectively turned on or off depending on whether atrial tachycardia is present. An atrial tachycardia condition is recognized by analyzing a pattern of short and long atrial event intervals. The pattern may be changed by adjusting a pacing parameter such as a maximum ventricular pacing rate. The pattern changes because by changing the pacing parameters, some of the atrial events are uncovered which have been previously hidden or masked by blanking periods, especially the cross channel atrial blanking period.

US 5,623,936 discloses an "VF immune" cardiac event detector for an implantable medical device. The detector consists of high-frequency and a low-frequency delta converters coupled to an intra-cardiac signal. The high-frequency delta converter is tuned to be responsive to R-waves and VF waveforms. However, the low-frequency delta converter is tuned to be responsive only to VF waveforms. By monitoring the difference between the outputs of the high-frequency and the low-frequency delta converters, the system can effectively discriminate between "true" R-waves and VF waveforms.

### Summary

According to aspects of the present disclosure, a user-adjustable external cardiac pacemaker and a method of using the adjustment information output by the user-adjustable external cardiac pacemaker are provided according to the independent claims. Preferred embodiments are recited in the dependent claims.

According to a first example not forming part of the invention, a method of controlling operation of a user-adjustable cardiac pacemaker, or a software model thereof, in a training mode is provided. The method comprises generating at least one cardiac event by a feedback-based heart simulator, wherein the cardiac event includes a pulse parameter set defining at least a pulse amplitude. The method further comprises transmitting the at least one cardiac event, or a first signal representation derived therefrom, to an electro cardiogram, ECG, generator configured to generate ECG graphics triggering a user to perform, in the training mode, one or more user adjustments at the cardiac pacemaker or its software model. Further still, the method comprises transmitting the at least one cardiac event, or a second signal representation derived therefrom, to the cardiac pacemaker or its software model, and acquiring stimulus information on one or more stimuli generated by the cardiac pacemaker or its software model. In the method presented herein, the stimulus information is fed back to the heart simulator for use in generation of one or more further cardiac events.

The cardiac pacemaker is an external pacemaker.The exemplary software model, which is not forming a part of the invention, may model operation of the external pacemaker or of an internal pacemaker.

The heart simulator may be configured to operate in real-time. The heart simulator may be part of an embedded system.

The user adjustments may occur at a user interface provided at the pacemaker itself. The user interface may be a mechanical user interface. The mechanical user interface may comprise one or more dials, one or more switches or one or more buttons. In the case of an internal pacemaker, which is an exemplary case not forming part of the invention, the user adjustments may occur at a user interface that is provided remotely from the internal pacemaker (e.g., using a computer coupled to the internal pacemaker via a wireless transmission link). In the case of a software model, the user adjustments may occur at a software-based user interface (e.g., at a graphical user interface, or GUI, that may optionally be provided at a touchscreen).

The method may comprise receiving, from the cardiac pacemaker or its software model, adjustment information indicative of a user adjustment. As understood herein, the adjustment information indicative of a user adjustment also comprise the scenario a missing or outstanding user adjustment that would have been expected.

The adjustment information may generally be indicative of current pacemaker settings, wherein (lack of) a user adjustment can be determined by a (missing) change of the pacemaker settings over a period of time. The adjustment information may be pulled from the pacemaker (using, e.g., a request/response messaging scheme) or pushed by the pacemaker (e.g., in response to detection of a user adjustment by the pacemaker).

The adjustment information may be received in the form of a data set or a sequence of data sets. The adjustment information may received separately from the stimulus information. As such, the adjustment information may be different from the stimulus information.

The stimulus information may be acquired from analog stimuli output by the cardiac pacemaker. Additionally, or as an alternative, the adjustment information may be received as digital information from the cardiac pacemaker. As an example, an external pacemaker may have an analog interface configured to output the stimuli and a digital interface configured to output the adjustment information.

The adjustment information may be indicative of one or more adjustment parameters including a pacing rate, a capture threshold, a sensing threshold, an atrio-ventricular (AV) delay, and a pacing mode (e.g., selection of a particular mode or a mode change). The adjustment information may comprise a time stamp. For example, each individual adjustment parameter may be associated with a time stamp in the adjustment information.

The method may comprise evaluating the adjustment information with respect to one or more of an expected user adjustment and the at least one cardiac event. The method may further comprise generating user training information indicative of a result of the evaluation. As an example, the heart simulator may be configured to execute a training program simulating the at least one (e.g., pathologic) cardiac event that is associated with one or more expected user adjustments (e.g., over a certain period of time), and the adjustment information may be evaluated to determine deviations between the user adjustments comprised in the adjustment information and the expected user adjustments (using, e.g., thresholding decisions). Such deviations may then form the basis of the user training information that may be output to the user.

The at least one cardiac event may be transmitted to at least one of a user training application and the ECG generator in association with a time stamp. The ECG generator may be configured to evaluate the time stamps associated with the cardiac events so as to properly align same in time in the (e.g., dynamically generated) ECG graphics. The user training application may be configured to evaluate the adjustment information based on the time stamp comprised therein and the time stamp associated with the at least one cardiac event. The timing basis (e.g., clock) used for generating the time stamp associated with the cardiac event may be the same as, or may be synchronized with, the timing basis for generating the time stamp associated with adjustment parameter(s) in the adjustment information.

The ECG generator may be comprised by the apparatus or by a training system also comprising the apparatus. In such a case, the method may further comprise calculating, by the ECG generator, a heart rate from at least the set of cardiac events. Optionally, the method may further comprise incorporating, by the ECG generator, the heart rate in the ECG graphics for presentation to the user.

The method may comprise converting the pulse parameter set into at least one of the first and the second signal representation. The first and/or second signal representation may be time-varying and representative of the pulse amplitude. The first and/or second signal representation may be indicative of at least one of a pre-defined pulse duration and a pre-defined pulse shape.

The pre-defined pulse shape may have any form. It may be a physiological pulse shape or a geometrically defined pulse shape. The pulse shape may conform to a test signal generator signal as defined in ISO 14708-2:2019-09.

The pulse shape may be triangular with a rising flank and a falling flank. As an option, the rising flank may be steeper than the falling flank. It has been found that triangular (and other geometrically defined) pulse shapes can electronically be generated, using suitable software and/or hardware, at a very fast rate, thus allowing an efficient real time training in regard to highly dynamic cardiac events (e.g., representative of fast and complex arrhythmia).

The ECG graphics may be configured to visualize one of the triangular pulse shape and a superposition of multiple triangular pulse shapes (occurring, e.g., in a temporally close relationship). It has been found that a geometrical simplification of physiological pulse shapes enhances the training efficiency as the user can, for example, concentrate on the temporal relationship between successive cardiac events without being distracted by physiological details.

The method may comprise causing the heart simulator to generate the at least one cardiac event so as to trigger the one or more user adjustments at the cardiac pacemaker or its software model. The at least one cardiac event may be indicative of a pathologic behavior of the simulated heart, such as conduction abnormalities or irregular heart beats. Conduction abnormalities include all forms of AV blocks, and irregular heart beats include atrial fibrillation.

The at least one cardiac event may be generated by the heart simulator under control of a training program defining the at least one cardiac event. As such, the at least one cardiac event may be at least partially defined by a training program that controls operation of the heart simulator. In addition, or as an alternative, to defining the at least one cardiac event by a training program, the at least one cardiac event may be at least partially defined by control information that is received in (e.g., in real-time) via a control interface. The control interface may be configured to communicate with (e.g., control) the heart simulator. The control interface may be provided at the apparatus. The control interface of the apparatus may be configured to be coupled (i.e., extended) to an external control interface of a computer operated by a trainer (i.e., an instructor) during a training session. The external control interface may comprise a GUI.

The at least one cardiac event may at least partially be generated, or defined, in response to the adjustment information. As an example, the training program may be configured as being responsive to the adjustment information. As a further example, the adjustment information may be output to the trainer (e.g., at a display device) who then may define the control information correspondingly.

The heart simulator may be configured to generate the at least one cardiac event based on one or more of the following possibly variable parameters: a capture threshold; one or more of a P, Q, R, S, and T wave peak amplitude; atrial fibrillation; retrograde conduction; prolonged AV delay; AV block; intrinsic heart rhythm; intrinsic heart rate; abnormal conduction from or through the atrioventricular node (AV node); atrio-ventricular disassociation. One or more of those parameters may be controllable by the training program or via the control interface.

The pulse parameter set may further define an event type identifier associated with the pulse amplitude. The event type identifier may be indicative of the cardiac event being of a ventricular event type or an atrial event type. In some variants, the ventricular event type may distinguish between a left ventricle and a right ventricle.

The stimulus information may be indicative of one or more of a signal amplitude and, optionally, an associated heart chamber identifier. The heart chamber identifier may be indicative of an atrium or of a ventricle. In some variants, the heart chamber identifier may additionally distinguish between a left ventricle and a right ventricle.

Also provided is computer program product comprising program code portions that cause at least one processor to execute the steps of any of the preceding claims when the computer program product is executed by the at least one processor. The computer program product may be stored on a computer-readable recording medium, such as a CD-ROM, a semiconductor memory, and so on. Moreover, the computer program product may be provided for download or may be hosted in a computing cloud.

A variant is directed to a training system comprising the user-adjustable cardiac pacemaker and an apparatus for controlling operation of the user-adjustable cardiac pacemaker in a training mode. The apparatus is configured to generate at least one cardiac event by a feedback-based heart simulator, wherein the cardiac event includes a pulse parameter set defining at least a pulse amplitude. The apparatus is further configured to transmit the at least one cardiac event, or a first signal representation derived therefrom, to an electro cardiogram, ECG, generator configured to generate ECG graphics triggering a user to perform one or more user adjustments at the cardiac pacemaker in the training mode. Further still, the apparatus is configured to transmit the at least one cardiac event, or a second signal representation derived therefrom, to the cardiac pacemaker. Moreover, the apparatus is configured to acquire stimulus information on one or more stimuli generated by the cardiac pace-maker to feed the stimulus information back to the heart simulator for use in generation of one or more further cardiac events.

The apparatus may be configured to perform any of the method steps presented herein.

Moreover, an aspect of the present disclosure relates to a user-adjustable external cardiac pacemaker comprising a first input interface configured to receive a signal representation of a cardiac event having a pulse amplitude, a first output interface configured to output one or more stimuli generated by the cardiac pacemaker, and a second output interface configured to output adjustment information indicative of a user adjustment.

The external cardiac pacemaker may further comprise a user interface for performing user adjustments controlling operation of the pacemaker, and electric circuitry (including, e.g., a processor) for transforming the user adjustments into the (optionally time-stamped) adjustment operation. The user interface may comprise mechanical components such as one or more dials, one or more switches or one or more buttons. Additionally, or as an alternative, the user interface may comprise a touchscreen.

The first output interface may be an analog interface. The first output interface may be configured to be coupled to a patient, to the apparatus taught herein and/or to a training system comprising the apparatus taught herein. The second output interface may be a digital interface. The second output interface may be configured to be coupled to the apparatus taught herein and/or to a training system comprising one or more of a computer and the apparatus taught herein.

### Brief Description of the Drawings

Further aspects, details and advantages of the present disclosure will become apparent below from a detailed description of exemplary embodiments and examples and from the drawings, wherein:
- Fig. 1: is a diagram illustrating a training system comprising an embodiment of an external cardiac pacemaker of the present disclosure;
- Fig. 2: is a block diagram illustrating an apparatus;
- Fig. 3: is a flow diagram of an exemplary method;
- Figs. 4 - 6: are schematic diagrams illustrating exemplary apparatus variants;
- Figs. 7 - 9: are schematic illustrations of pulse shape embodiments;
- Fig. 10: is a schematic diagram illustrating exemplary aspects of a more detailed apparatus;
- Fig. 11: is a schematic illustration of a heart simulator;
- Figs. 12 -14: are schematic illustrations of training system set-ups; and
- Fig. 15: is a schematic illustration of a control interface for a heart simulator.

### Detailed Description

In the following description, for purposes of explanation and not limitation, specific details are set forth in order to provide a thorough understanding of the present disclosure. It will be apparent to one skilled in the art that the present disclosure may be practiced in other embodiments that depart from these specific details.

Those skilled in the art will further appreciate that the steps, services and functions explained herein may be implemented using individual hardware circuits, using software functioning in conjunction with a programmed microprocessor or general purpose computer, using one or more application specific integrated circuits (ASICs) and/or using one or more digital signal processors (DSP). It will also be appreciated that when the present disclosure is described in terms of a method, it may also be embodied in one or more processors and one or more memories coupled to the one or more processors, wherein the one or more memories store one or more computer programs that cause the one or more processors to perform the steps, services and functions disclosed herein when executed by one or more processors.

In the following description of examples and exemplary embodiments, the same reference numerals denote the same or similar components.

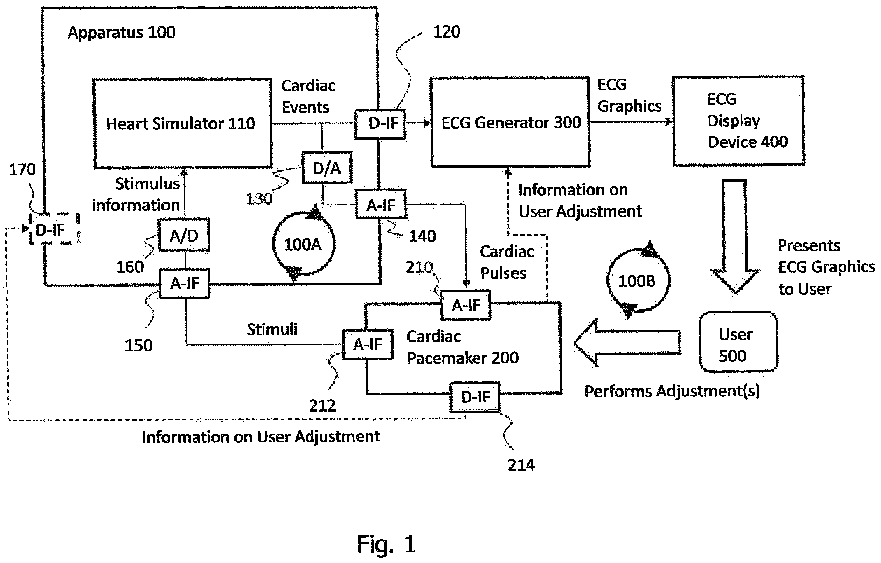

Fig. 1 illustrates a training system comprising an apparatus 100 for controlling operation of a user-adjustable cardiac pacemaker 200 that is also comprised by the training system. The pacemaker 200 is realized as an external pacemaker.

The training system further comprises an ECG generator 300 configured to generate a ECG graphics for presentation by an ECG display device 400 to a user 500 to be trained. In some variants, the ECG generator 300 is integrated into the apparatus 100. In other variants, the ECG generator 300 is a device co-located with the apparatus 100 or located remotely therefrom. The ECG generator 300 may in particular be configured to generate a surface ECG.

As illustrated in Fig. 1, the apparatus 100 comprises a heart simulator 110 that is configured to generate digital cardiac events. Each cardiac event may at least be indicative of a pulse amplitude and, optionally, one or more further pulse parameters. The pulse amplitude may be indicated in mV. In some implementations, the apparatus 100 is realized as an embedded platform into which the heart simulator 110 is integrated. The implementation as an embedded platform permits an efficient real-time operation of the apparatus 100 in an inner feedback loop 100A with the pacemaker 200 and an outer feedback loop 100B with the user 500.

The heart simulator 110 may be implemented as a dedicated digital hardware component (e.g., with a dedicated processor and a dedicated memory that stores program code causing the processor to simulate certain functions of a human heart). The heart simulator 110 offers settings and configurations that can be controlled in run-time by one or both of a suitable training program and a control interface (not shown).

The apparatus 100 further comprises a digital output interface 120 configured to output the cardiac events generated by the heart simulator 110 to the ECG generator. The digital output interface 120 may conform to a Universal Serial Bus (USB) standard or any other standard for digital data communication. The apparatus 100 further comprises a digital/analog (D/A) converter 130 configured to convert the digital cardiac events generated by the heart simulator 110 into an analog signal representation in the form of time-varying cardiac pulses. Those cardiac pulses can be output via an analog output interface 140 of the apparatus 100 to the pacemaker 200. The analog output interface 140 can be realized using a mechanical plug connection.

The pacemaker 200 is configured to receive the cardiac pulses at an analog input interface 210. From the perspective of the pacemaker 200, those cardiac pulses are generated by a human heart and received via corresponding sensing leads that are normally electrically coupled to the analog interface 210 and configured to be suitably placed to detect a patient's heart activity. In case the pacemaker 200 is configured as an external pacing device, those sensing leads are commonly transvenous pacing leads or surgical pacing wires (also referred to as heart wires. In other variants, the sensing leads can be realized as patches configured to be placed on a patient's skin surface (e.g., in case of transthoracic pacing). The sensing leads may also serve as leads for stimulation. In the presently described training scenario, the sensing leads are coupled to the analog output interface 140 using an optional adapter (or replaced by a cable extending from the analog output interface 140 of the apparatus 100 to the analog input interface 210 of the pacemaker 200). In an exemplary case not forming a part of the invention, the pacemaker 200 is an internal pacing device configured to be implanted, its sensing leads are configured to be implanted as well. In the presently described training scenario, such an internal pacemaker 200 will, of course, not be in an implanted state, so that its sensing leads can easily be coupled, optionally via a suitable adapt-er, to the analog output interface 140.

The pacemaker 200 is configured to generate electric stimuli. Those stimuli may at least in part be generated responsive to the cardiac pulses output by the apparatus 100 and sensed by the pacemaker 200. From the perspective of the pacemaker 200, those stimuli are output to a human heart via an analog output interface 212 to which corresponding stimulation leads can electrically be coupled. The simulation leads are configured to be suitably placed to stimulate a patient's heart activity. In case the pacemaker 200 is configured as an external pacing device, those stimulation leads are commonly transvenous pacing leads or surgical wires, but they may also be realized as patches configured to be placed on a patient's skin surface. The stimulation leads may also serve as leads for sensing. In the presently described training scenario, the stimulation leads are coupled to an analog input interface 150 of the apparatus 100 using an optional adapter (or replaced by a cable extending from the analog output interface 212 of the pacemaker 200 to the analog input interface 150 of the apparatus 100). In an exemplary case not forming a part of the invention, the pacemaker 200 is an internal pacing device con-figured to be implanted, its stimulation leads are configured to be implanted as well. In the presently described training scenario, the internal pacemaker 200 will evident-ly not be in an implanted state, so that its stimulation leads can easily be coupled, optionally via a suitable adapter, to the analog input interface 150.

In some variants, the two analog interfaces 140, 150 on the side of the apparatus are integrated into a single analog input/output interface. In the exemplary case in which the pacemaker 200 is realized as a software model external or internal to the apparatus 100, which is not forming a part of the invention, those analog interfaces 140, 150 can be replaced by at least one digital interface or an Application Programming Interface (API).

As illustrated in Fig. 1, the apparatus 100 comprises an analog/digital (A/D) converter 160 coupled between the analog input interface 150 and the heart simulator 110 to close the feedback loop 100A. The A/D converter 160 converts the analog electric stimuli output by the pacemaker 200 into a digital signal representation comprising stimulus information indicative of the electric stimuli. In general, the stimulus infor-mation will at least be indicative of a pulse amplitude per detected electric stimulus.

As has been explained above, the pacemaker 200 is adjustable by the user 500 to control pacemaker operation in accordance with a patient's needs. In the present training scenario, those needs may be indicated to the user 500 by one or more cardiac events generated by the heart simulator 110 and visualized at the display device 400 to trigger one or more user adjustments at the pacemaker 200 (see Fig. 1). Depending on the pacemaker type, exemplary user adjustments at the pacemaker 200 comprise, but are not limited to, one or more of a pacing rate adjustment, a capture threshold adjustment, a sensing threshold adjustment, an adjustment of an atrio-ventricular delay, and a pacing mode adjustment. A pacing mode adjustment comprises adjustment of a dedicated mode or switching form one mode to another mode (e.g., from an x-channel mode to an y-channel mode, with x ≠ y).

In the case of an external pacemaker 200, the user adjustments may directly be performed at a mechanical user interface of the pacemaker 200 (e.g., at one or more dials, one or more switches or one or more buttons). In the exemplary case of an internal pacemaker 200, which is not forming a part of the invention, the user adjustments may be performed at a dedicated control device (not shown in Fig. 1) and may wirelessly be communicated to the pacemaker 200. The control device may take the form of a computing device (e.g., a tablet or personal computer) having a graphical user interface permitting the user 500 to input the desired adjustments.

The pacemaker 200 may in some implementations be configured to generate adjustment information indicative of the user adjustments. The user adjustment infor-mation may take the form of a parameter set indicative of a parameter type and, optionally, an associated time stamp indicative of when the adjustment was made. The parameter set may further comprise a value indicative of (e.g., quantifying) the user adjustment. As such, the parameter set may for at least some user adjustments comprise a type/value (T/V) pair with an associated time stamp.

In the case of an external pacemaker 200, the adjustment information is output via an interface 214 of the pacemaker 200, which may be a digital interface, to one or both of the apparatus 100 and the ECG generator 300 (or to a dedicated training sub-system), as indicated by dashed lines in Fig. 1. As also shown in dashed lines in Fig. 1, the apparatus 100 may comprise a dedicated digital interface 170 configured to receive the adjustment information from the external pacemaker 200. The adjustment information will thus be received by the apparatus 100 separately from the stimuli and the associated stimulus information. In the exemplary case of an internal pacemaker 200, which is not forming a part of the invention, the adjustment infor-mation may wirelessly be communicated from the internal pacemaker 200 to the dedicated control device that was used to input the user adjustments.

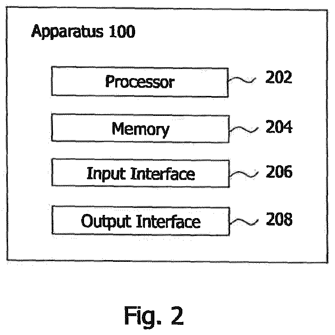

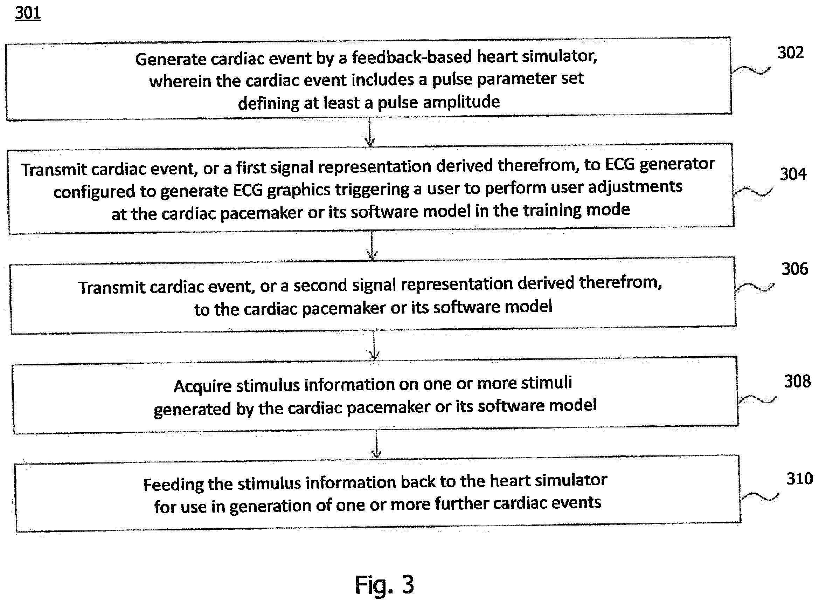

In the following, an exemplary implementation of the apparatus 100 of Fig. 1 will be described with reference to Fig. 2, and general operation of the apparatus 100 will be described with reference to a method example illustrated in flow diagram 301 of Fig. 3.

In the apparatus illustrated in Fig. 2, the apparatus 100 comprises a processor 202 and a memory 204 coupled to the processor 202. The memory 204 stores program code (e.g., in the form of a set of instructions) that controls operation of the processor 202 so that the apparatus 100 is operative to perform any of the method aspects presented herein. As understood herein, a processor, such as pro-processor 202, may be implemented using any processing circuitry and is not limited to, for example, a single processing core but may also have a distributed topology (e.g., using cloud computing resources).

The apparatus 100 of Fig. 2 further comprises at least one input interface 206 and at least one output interface 208. The at least one input interface 206 may be configured to implement the interfaces 150, 170 described above with reference to Fig. 1. The at least one output interface 208 may be configured to implement the interfaces 120, 140 described above with reference to Fig. 1. In some variants, the apparatus 100 may further comprise analog electronics not illustrated in Fig. 2.

The heart simulator 110 described above with reference to Fig. 1 may be realized using program code stored in the memory 204 and executed by the processor 200, or may alternatively be realized on a dedicated sub-platform integrated into the apparatus 100.

Now referring to the flow diagram 301 of Fig. 3, operation of the apparatus 100 comprises a step 302 of generating at least one cardiac event by the feedback-based heart simulator 110. The cardiac event includes a pulse parameter set defining at least a pulse amplitude of a cardiac pulse to be output via the analog output interface 140 to the pacemaker 200. In some variants, the heart simulator 110 is configured to generate in parallel atrial cardiac events and ventricular cardiac events (in accordance with two dedicated logical event channels). In other variants, the heart simulator is configured to generate in parallel atrial cardiac events and ventricular cardiac events for the left ventricle and the right ventricle (in accordance with three dedicated logical event channels). The pulse parameter set will then further define an event type identifier associated with the pulse amplitude. The event type identifier is indicative of the cardiac event being of a ventricular event type or an atrial event type.

The method further comprises a step 304 of transmitting the at least one cardiac event, or a first signal representation derived therefrom (e.g., a time-varying signal representation), to the ECG generator 300. As explained above, the ECG generator 300 is configured to generate ECG graphics for visualization by the display device 400. The visualized ECG graphics trigger the user 500 to perform, in a training mode, one or more user adjustments at the cardiac pacemaker 200 (or its software model), such as one or more of a pacing rate adjustment, a capture threshold adjustment, a sensing threshold adjustment, and a pacing mode adjustment

Further still, the method comprises a step 306 of transmitting the at least one cardiac event, for example in the form of an analog signal representation generated by the D/A converter 130, via the analog output interface 140 to the cardiac pacemaker 200. In the case a software model implementation of the pacemaker 200, no such D/A conversion will of course be required.

In step 308, the apparatus 100 acquires stimulus information on one or more electric stimuli generated by the cardiac pacemaker 200 (or its software model). Acquisition of the stimulus information comprises receipt of the stimuli in analog form by the apparatus 100 via the analog input interface 150 and conversion of the analog stimuli by the A/D converter 160 into a digital format representative of the stimulus infor-mation. The stimulus information is indicative of at least one signal amplitude.

In a two channel scenario, the electric stimuli are generated separately by the pace-maker 200 for an atrium and a ventricle of a patient's heart to be applied via dedicated stimulation leads. In this case, the analog interface 150 may also include two dedicated channels (e.g., in the form of two dedicated electrical contacts). Similar considerations apply for a 3-channel or a 4-channel scenario. The stimulus infor-mation generated by the A/D converter 160 will thus comprise an associated heart chamber identifier per signal amplitude. The heart chamber identifier is indicative of a particular signal amplitude being intended to be applied to the atrium of the ventricle.

In step 310 of the method illustrated in Fig. 3, the stimulus information is fed back to the heart simulator 110 for use in generation of one or more further cardiac events. The heart simulator 110 is thus configured to process the stimulus information in a feedback-based manner in the cardiac event generation process.

In an optional step not illustrated in Fig. 3, the apparatus 100 further receives, via the optional digital interface 170, the adjustment information as generally described above with reference to Fig. 1 (see the dotted lines in Fig. 1). In certain variants, the adjustment information may additionally, or alternatively, be transmitted by the pacemaker 200 to any other system component illustrated (or not illustrated) in Fig. 1. In all those variants, the adjustment information thus received is generally processed to generate a feedback to the user 500 as to whether or not the user adjustments were successful in accordance with a pre-defined training objective (e.g., tackling simulated arrhythmia).

The external cardiac pacemaker 200 presented herein may be configured to output the adjustment information in a push-based manner (e.g., in regular intervals or upon detection that a user adjustment has actually been performed). Additionally, or as an alternative, the adjustment information may be output by the pacemaker 200 in a pull-based manner (e.g., in a response message that is generat-ed by the pace-maker 200 upon receipt of a request message). The adjustment infor-mation may comprise a data set generally indicative of the current setting(s) of one more adjustable parameters of the pacemaker 200 or specifically indicative of a par-ticular parameter adjustment performed by the user 500. In the first case, a particu-lar parameter adjustment may be derived from a change in the current parameter setting(s) from one data set to a subsequent data set.

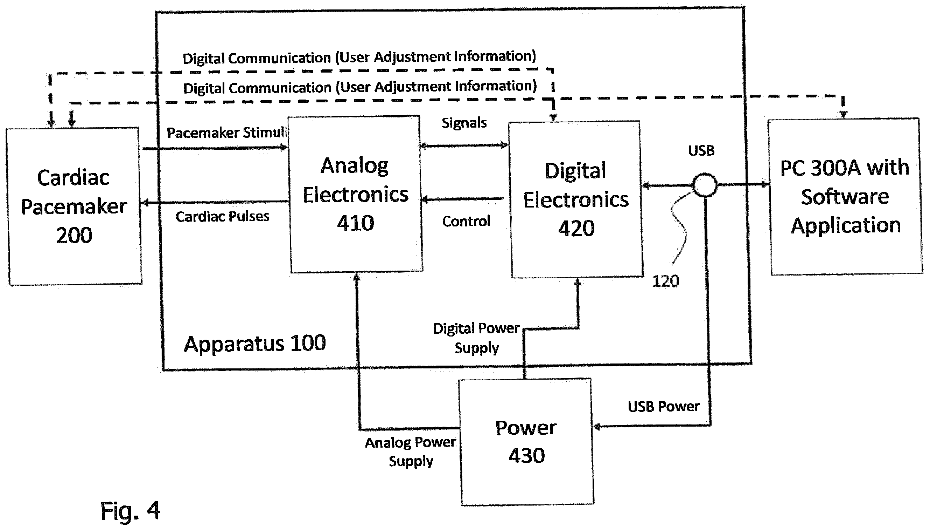

Fig. 4 illustrates further aspects of the system illustrated in Fig. 1 and in particular of the apparatus 100. Most of the interfaces shown in Fig. 1 have been omitted for ease of illustration.

As shown in Fig. 4, the apparatus 100 comprises analog electronics 410 and digital electronics 420 powered by a power supply 430. The ECG generator 300 is exemplarily configured as a personal computer (PC) 300A coupled to the apparatus 100 via the interface 120 (here: a bi-directional USB interface that additionally powers the power supply 430). The PC 300A hosts a software application that is programmed to generate ECG graphics from cardiac events generated by and received from the apparatus 100. The ECG graphics will then be output to the user 500 via the display device 400 of Fig. 1.

The digital electronics 420 of Fig. 4 contain an embedded software-programmable microprocessor system or programmable digital logic implementing the heart simulator 110 of Fig. 1 to permit real-time operation of the heart simulator 110 in the feedback loop 100A with the cardiac pacemaker 200. This real-time operation permits a realistic simulation of a real heart based on feedback loop from a real pace-maker 200 and, thus, a more efficient user training.

The digital electronics 420 exchange digital signals with the analog electronics 410 (which, therefore, comprise the D/A converter 130 and the A/D converter 160 of Fig. 1) and control operation thereof. The electronic signals comprise the signaling of cardiac events to the analog electronics 410 and the signaling of stimulus infor-mation to the digital electronics 420, as generally explained above. Control of the analog electronics 410 by the digital electronics 420 comprise electric load switching.

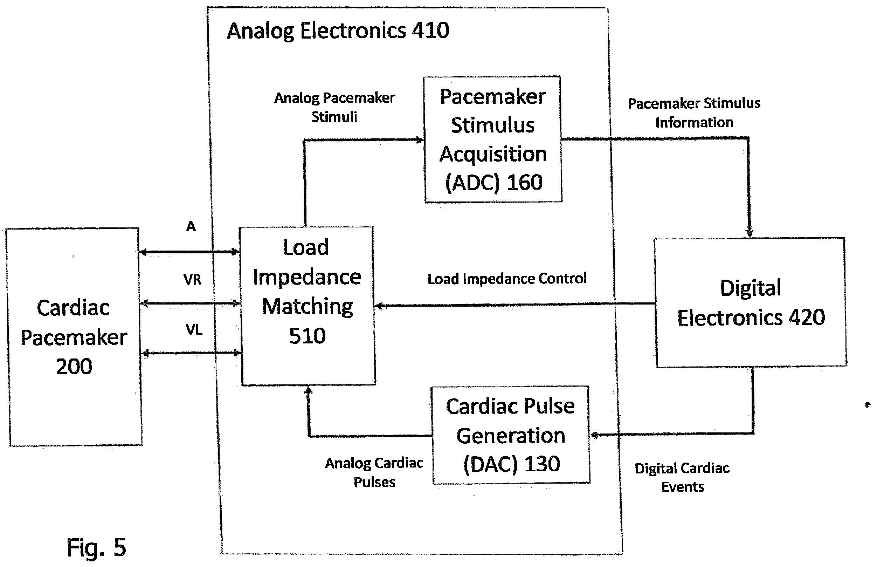

Now further referring to Fig. 5, central components of the analog electronics 410 are illustrated. The analog electronics 410 comprise the D/A converter 130 of Fig. 1 to convert a time-varying digital cardiac event signal (or a sequence of discrete cardiac event data sets) from the digital electronics 420 into the cardiac pulses (as time-varying analog signals). The analog electronics 410 further comprise the A/D converter 160 of Fig. 1 to convert a time-varying analog stimuli signal from the pace-maker 200 into the stimulus information (as sequence of discrete data sets). The analog electronics 410 also comprises a component 510 configured for load impedance matching between the pacemaker 200 on the one side and the apparatus 100 on the other side. As shown in Fig. 5, the load impedance matching component 510 is controllable by the digital electronics 420 (e.g., so that the apparatus 100 can be used with different types of pacemakers 200).

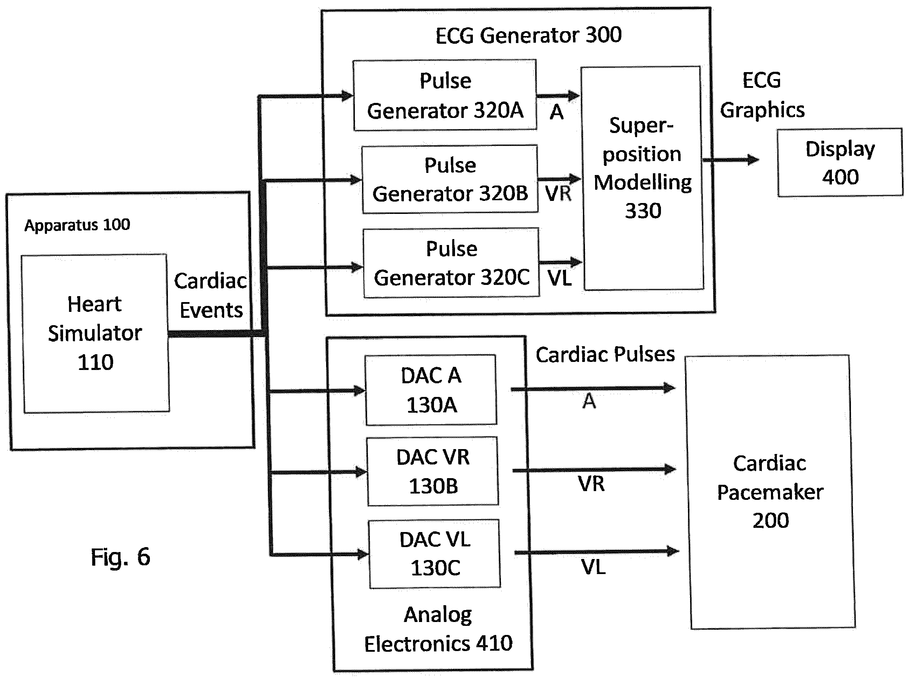

Fig. 5 exemplarily illustrates a 3-channel pacemaker 200 having dedicated sensing and stimulation leads for the atrium (A), the right ventricle (VR) and the left ventricle (VL). This means that dedicated cardiac events for the atrium, the left ventricle and the right ventricle are generated in parallel by the heart simulator 110 comprised by the digital electronics 420, as shown in Fig. 6, and corresponding stimuli are generated in parallel by the pacemaker 200.

Fig. 6 further illustrates that in a 3-channel scenario, the D/A converter 130 of Figs. 1 and 5 comprises three dedicated D/A converters 130A, B, C (i.e., one per channel A, RF, LV). In a similar manner, the ECG generator 300 comprises three dedicated pulse generators 320A, B, C (i.e., one per channel A, RV, LV). Those pulse generators 320A, B, C will in parallel generate a dedicate ECG pulse per cardiac event and channel (in either parameterized form or as partial graphics). Those three individual "in-tercardial" ECG (IECG) pulses are then superimposed and modelled by a superposition component 330 to generate the ECG graphics (e.g., simulating a surface ECG) for a particular period of time (as defined, e.g., by a simulated heart rate). Successive ECG graphics for successive periods of time will then be continuously visualized on the display device 400. It will be appreciated that the scenario of Fig. 6 could easily be modified for a 2-channel scenario with an A channel and a V channel only, of for any other number of channels. In a similar manner, the scenario of Fig. 6 could easily be modified for a 3-channel scenario encompassing right atrium (AR), left atrium (AL) and a single ventricle (VR or VL), or for any other number of channels.

In some variants, each of the pulse generators 320A, B, C is configured to generate a geometrically defined pulse shape. A geometrically defined pulse shape may be characterized by a limited number of pulse parameters per cardiac event, including a pulse amplitude that may vary from one cardiac event to the subsequent cardiac event on the same channel. Compared to physiologically "correct" pulse shapes, geometrically defined pulse shapes can be generated faster by the ECG generator 300 and are easier to cognitively "process" by the user 500 when visualized on the display device 400. The latter aspect is particularly beneficial in the training scenario described herein.

The pulse shape may be triangular with a rising flank in a temporal direction towards a peak defined by the pulse amplitude and a falling flank behind that peak. In some variants, a temporal length of the pulse may be pre-defined (e.g., fixed), or it may alternatively be signaled as a further pulse parameter. In a similar manner, a temporal length of at least one of the rising flank and the falling flank may be pre-defined (e.g., fixed), or it may alternatively be signaled as a further pulse parameter.

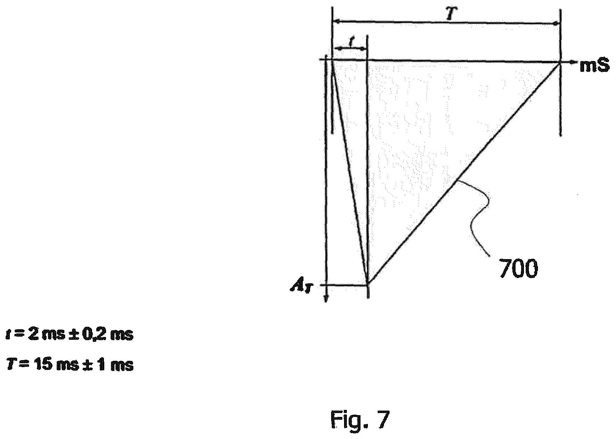

An exemplary triangular pulse 700 with an overall duration T, a rising flank having a duration t, a peak defined by a pulse amplitude A_{T}, and a falling flank having a duration T - t is illustrated in Fig. 7. The duration t of the rising flank and the overall duration T of the pulse are fixed in the millisecond regime, as indicated in Fig. 7. In this way, the number of pulse parameters to be communicated to the ECG generator 300 is reduced to the pulse amplitude, and ECG graphics generation by the ECG generator 300 can be accelerated. In some variants, the triangular pulse shape may conform to a test signal generator signal as defined in ISO 14708-2:2019-09.

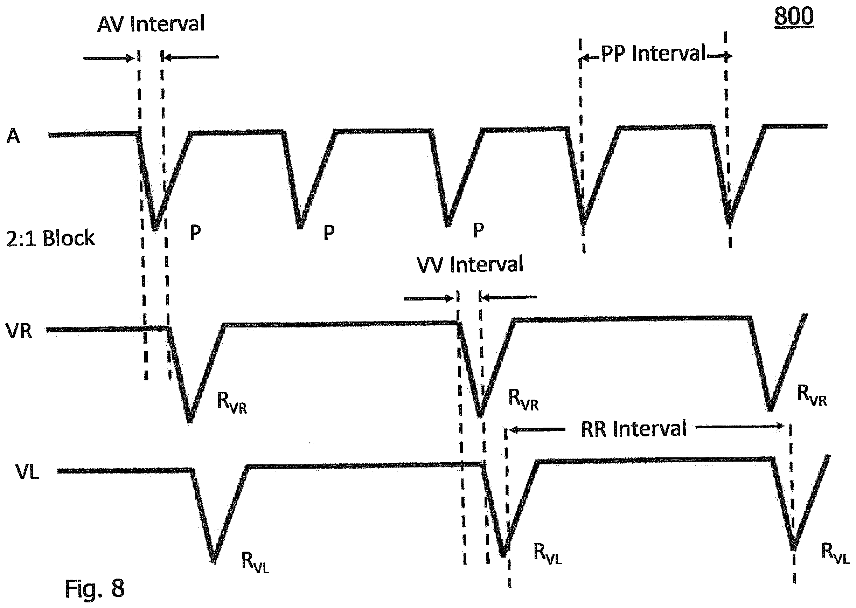

Fig. 8 illustrates in a time diagram 800 the parallel generation of pulses by the ECG generator 300 in the 3-channel scenario of Fig. 6. As becomes apparent from Fig. 6, the individual pules have triangular pulse shapes.

In the simulated atrial signal (A) of Fig. 8, each peak represents a so-called P wave pulse amplitude. The atrial pulse rate is Rate_{A}, = 60/PP_Interval (in bpm), wherein the PP_Interval is the interval between successive P waves due to atrial depolarization. In the simulated right ventricular signal (VR), each peak represents a so-called R wave pulse amplitude, and similar for the left ventricular signal (VL). The ventricular pulse rate is Rate_{V} = 60/RR_Interval (in bpm), wherein the RR_Interval is the interval between successive R waves. The ventricular pulse rate Rate_{V} may be equal to the atrial pulse rate Rate_{A} or less in case of a so-called 2:1 block. An AV interval is defined as the delay between the atrial signal and the ventricular signal (in Fig. 8: for the right ventricle). A VV interval is defined as the delay between the signal of the right ventricle and the signal of the left ventricle.

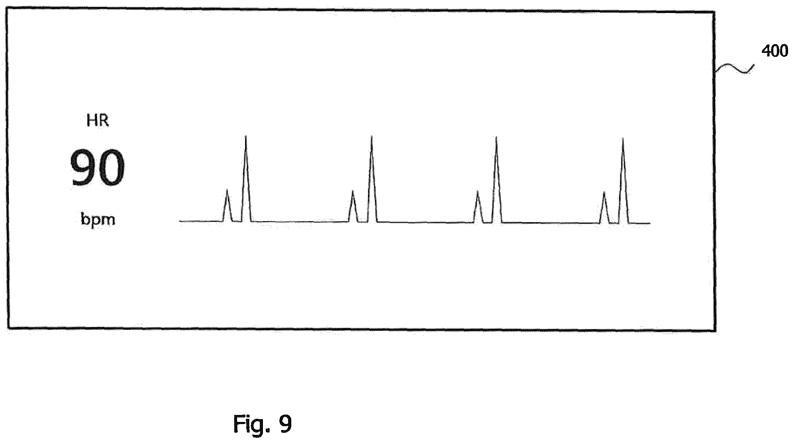

Fig. 9 illustrates the resulting superposition of triangular pulses as visualized by the display device 400 for a 2-channel scenario. The smaller triangular pulses visualize P waves, whereas the larger triangular pulses visualize R waves. Also the heart rate will be visualized by the display device (as a numerical value in bpm). The heart rate will be calculated by the ECG generator 300 based on the cardiac events and the associated time stamps. A similar superposition and visualization as shown in Fig. 9 will be obtained in the 3-channel scenario of Figs. 6 and 8.

For properly visualizing the temporal relationship of the pulses on each channel and across different channels, the cardiac events are communicated from the apparatus 100 to the ECG generator 300 together with associated time stamps. The ECG generator 300 is configured to evaluate the time stamps associated with the cardiac events so as to properly align same in time in the dynamically generated ECG graphics (see Fig. 8). The time stamps may be generated by the apparatus 100 in different ways, for example by "packaging" a cardiac event output by the heart simulator 100 together with a time stamp freshly generated by the apparatus 100 (e.g., by a local clock operating on a millisecond scale) into a cardiac event message output to the ECG generator 300 (e.g., to an external computing device such as PC 300A programmed to emulate the ECG generator 300). In a 2-channel scenario, the cardiac event message will further comprise an event type identifier indicative of the cardiac event being of a ventricular event type or an atrial event type. In a 3-channel scenario (see Figs. 6 and 8), the ventricular event type will further distinguish between a left ventricle and a right ventricle. The event type identifier thus serves as a channel identifier for the ECG generator 300.

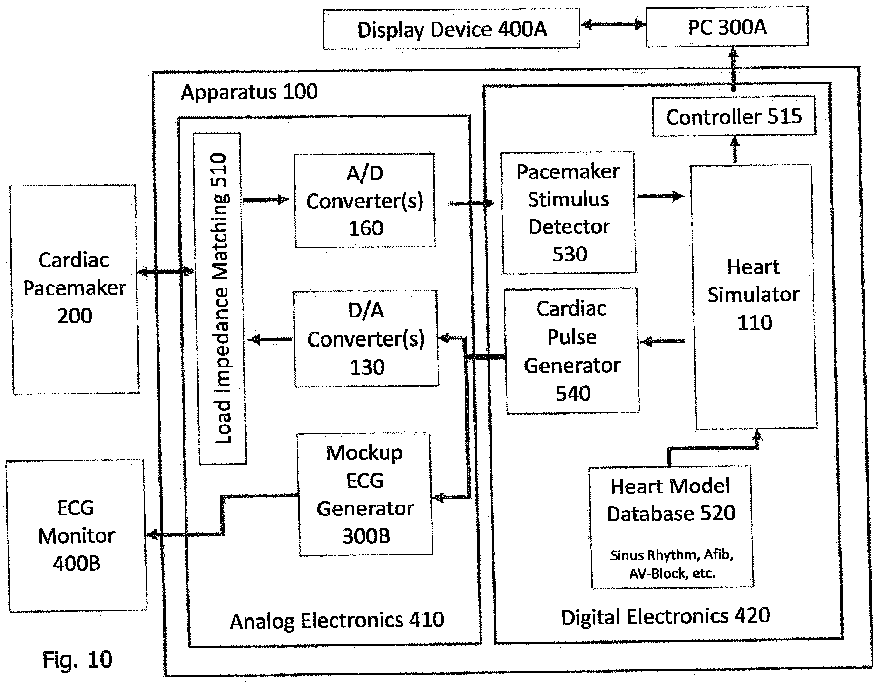

Fig. 10 is a schematic diagram illustrating a more detailed apparatus. The following description of Fig. 10 will focus on some additional system components such as a controller 515 as part of the digital electronics 420 of the apparatus 100.

The controller 515 interfaces the heart simulator 110 as well as the PC 300A with the ECG generator 300. The controller 515 is in charge of generating the cardiac event messages. To this end, the controller 515 associates each cardiac event output as pulse parameter set by the heart simulator 110 with a time stamp and, optionally, an event type identifier (i.e., channel identifier) and packages those data into the cardiac event message that is then output to the PC 300A. In one implementation, the pulse parameter set as generated by the heart simulator 110 will only include a single parameter indicative of a pulse amplitude (e.g., in mV). In another implementation, the pulse parameter set additionally comprises the event type identifier (so it will not need to be attributed by the controller 515).

The controller 515 in some variants also offers a control interface of the apparatus 100 for receiving, from the PC 300A or another computer operated by a trainer, control information. The control information controls operation of the heart simulator 110 (e.g., in reals time). As an example, the control information may be indicative of parameter settings to be applied by the heart simulator 110 for generation of the cardiac events.

As illustrated in Fig. 10, the digital electronics 420 further comprise a heart model database 520 with settings or configurations that define one or more training programs to control the heart simulator 110 in run-time. Possible control parameters that can be set or configured in this regard define a sinus rhythm, the AV block explained above with reference to Fig. 8, atrial fibrillation, and so on.

Still further, the digital electronics 420 comprise a pacemaker stimulus detector 530 coupled between A/D converter(s) 150 on the one hand and the heart simulator 110 on the other hand. It will be appreciated that there will generally be one A/D converter per pacemaker output channel (in a similar manner as illustrated for the three D/A converters 130A-C in Fig. 6). In some variants, the pacemaker stimulus detector 530 may be integrated into the heart simulator 110.

The pacemaker stimulus detector 530 is configured apply per channel one or multiple sensing thresholds to the digital output signals of the A/D converter(s) 150. A first sensing threshold per channel may relate to a minimum stimulus pulse amplitude (e.g., in terms of a measured voltage). An optional second sensing threshold may relate to a minimum stimulus pulse duration (e.g., in milliseconds). Both threshold types may be adjustable. The pacemaker stimulus detector 530 will only signal a stimulus included in the digital output signal of the A/D converter(s) 160 to the heart simulator 110 in case that stimulus is detected to satisfy the thresholding condition(s). Otherwise, the heart simulator 110 is not notified thereof. The stimulus information signaled to the heart simulator 100 for a detected stimulus include a detected stimulus pulse amplitude (e.g., in V) and an associated heart chamber identifier. In a 2-channel scenario, the heart chamber identifier is indicative of an atrium or of a ventricle. In a 3-channel scenario, the heart chamber identifier will additionally distinguish between a left ventricle and a right ventricle.

Further still, the digital electronics 420 comprise a cardiac pulse generator 540 coupled between the heart simulator 110 on the one hand and the D/A converter(s) 130 on the other hand. It will be appreciated that there will generally be one D/A converter 130 per pacemaker input channel (see the exemplary three D/A converters 130A-C in Fig. 6). In some variants, the cardiac pulse generator 540 may be integrated into the heart model 110.

The cardiac pulse generator 540 translates each cardiac event (i.e., each pulse parameter set) output by the heart simulator 110 into a time-varying digital signal indicative of a dedicated pulse shape. In this regard, a dedicated time-varying signal will be generated per channel (i.e., per D/A converter, see Fig. 6). The pulse shape may in particular be triangular, as illustrated in Fig. 7. The pulse shape generated by the cardiac pulse generator 540 need, however, not necessarily be the same pulse shape as the one that is to be visualized.

In case of a triangular pulse shape with a pre-defined pulse duration T and a pre-defined duration t of the rising flank (see Fig. 7), the pulse parameter set may just include the pulse amplitude A_{T} (and, optionally, the event type identifier) as input information for the cardiac pulse generator 540. In a 3-channel scenario, the digital output of the cardiac pulse generator 540 may have a temporal appearance similar to the scenario illustrated in Fig. 8. In view of the real-time operation of the apparatus 100, no time stamping is needed for continuous operation of the cardiac pulse generator 540.

The digital output generated by the cardiac pulse generator 540 per channel will not only be fed to the one or more D/A converters 130, but in parallel to an optional - ECG generator 300B coupled to a likewise optional ECG monitor 400B. The ECG monitor 400B is the "real" display device that will typically be used in connection with the "real" pacemaker 200 for patient treatment by the user 500 to be trained. Since the ECG monitor 400B typically has an analog input interface, the mock-up ECG generator 300B comprises one D/A converter per channel to convert the digital signal output by the cardiac pulse generator 540 into an analog ECG graphics signal the will trigger the ECG monitor 400B to display the ECG graphics to the user 500 that is to be trained. It will be appreciated that the mock-up ECG generator 300B and the ECG monitor 400B could be used in addition or as an alternative to the PC 300A and the display device 400A for user training. For example, in the scenario of Fig. 1, the mock-up ECG generator 300B and the ECG monitor 400B could be used to implement the ECG generator 300 and the display device 400, respectively.

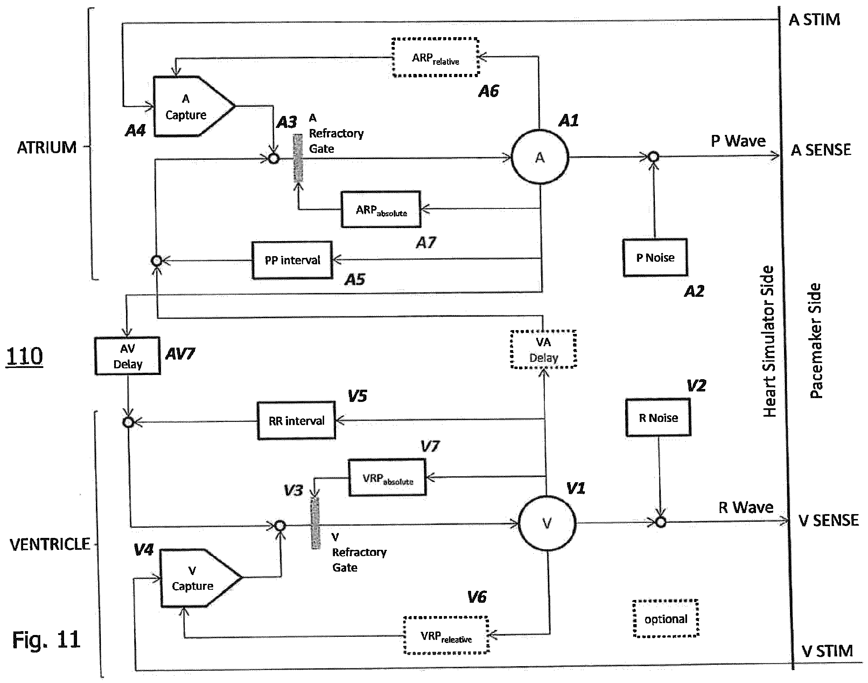

Fig. 11 is a schematic illustration of a 2-channel heart simulator. The heart simulator 110 has two logical inputs and two logical outputs differentiated based on the heart chamber identifier and event type identifier, respectively. As such, the heart simulator 110 of Fig. 11 has one input and one output per channel (atrial or "A" vs. ventricular or "V"). The inputs "A STIM" and "V STIM"" are coupled to the cardiac stimulus detector 530, while the outputs "A SENSE" and "V SENSE" are coupled to the cardiac pulse generator 540. The respective input and output infor-mation will generally be indicative of a respective pulse amplitude in combination with a channel identifier, as generally explained above.

The main operational mechanism of the heart simulator 110 is to produce atrial and/or ventricular heart muscle depolarizations (depicted by the round circles A and V; see reference numeral A1 and V1, respectively). For each channel, the depolarization is transformed into a pulse parameter indicative of voltage value that represents a pulse amplitude peak, which then may be detected by the attached pacemaker 200 as atrial or ventricular sense event, respectively. Optionally noise events A2, V2 can be added (e.g., at a certain interval per channel).

Each depolarization starts the absolute refractory period for the atrium (ARP) A7 and the ventricle (VRP) V7. As long as the absolute refractory period is not expired, all triggers (from whatever source) are ignored at an associated refractory gate A3, V3. The main heart oscillators are a PP timer A5 (aka. sinus node) and an RR timer V5. These timers realize what is known as heart rate. The heart simulator 110 can be triggered by the pacemaker output (A STIM or V STIM). However, any incoming pacemaker stimulation must pass the capture gate A3, A7. This means that the stimulation voltage must be above a capture threshold as defined by the cardiac stimulus detector 530. This capture threshold may be partly constant or may also depend on the time since the recent depolarization (by means of relative refractory period A6, V6). The atrial channel is linked with the ventricular channel by means of AV conduction AV7 which includes a certain delay (AV Delay).

The heart simulator 110 of Fig. 11 thus comprises multiple heart parameters that can be adjusted, for example to generate one or more cardiac events indicative of a pathologic symptom. Such cardiac events may be defined, via the heart parameters, using a training program (that may, e.g., be stored in the heart model database 520 of Fig. 10). Additionally, or as an alternative, such cardiac events may be defined, via the heart parameters, by control information manually configured by a human trainer via an external control interface (e.g., during run-time). As understood herein, the capture threshold(s) applied by the cardiac stimulus detector 530, and thus the cardiac stimulus detector 530 itself, are logically also comprised by the heart simulator 110.

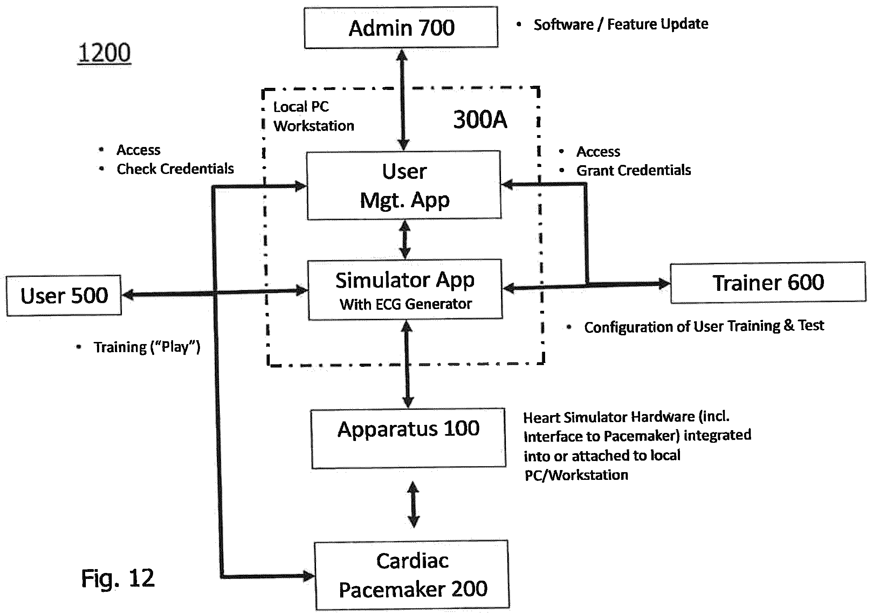

Figs. 12 to 15 are schematic illustrations of training system set-ups .

According to the set-up 1200 of Fig. 12, the PC 300A (see Fig. 10) or, alternatively, a workstation hosts a simulator application with the ECG generator 300 and, additionally, a control interface (implemented, e.g., as GUI) that permits a real-time training control by a trainer 600. In more detail, the control interface stretches from the PC 300A via the controller 515 to one or both of the heart simulator 110 and the heart model database 520 to allow an trainer adjustment of parameter settings or parameter configurations that control operation of the heart simulator 110 (see, e.g., the parameters discussed above in the context of Fig. 11). Moreover, the simu-lator application also permits an evaluation of user adjustments actually performed at the pacemaker 200. As explained above, corresponding user adjustment information may be received by the simulator application directly from the pacemaker 200 or from the apparatus 100 (see also dashed lines in Figs. 1 and 4). In this regard, the simulator application may evaluate the optionally time stamped user adjustments received from the pacemaker 200 with one or both of the optionally time stamped (i) information on expected user adjustments and (ii) cardiac events generated by the heart simulator 110 to check if the actual user adjustments correspond to a training goal

The PC 300A comprises a user management application that can be configured by an administrator 700 via a suitable interface. An exemplary configuration includes an update of the simulator application or of its features. The user management application is also in charge of access control in regard to both the user 500 and the trainer 600 (e.g., by checking corresponding credentials that need to be input by the user 500 and the trainer 600).

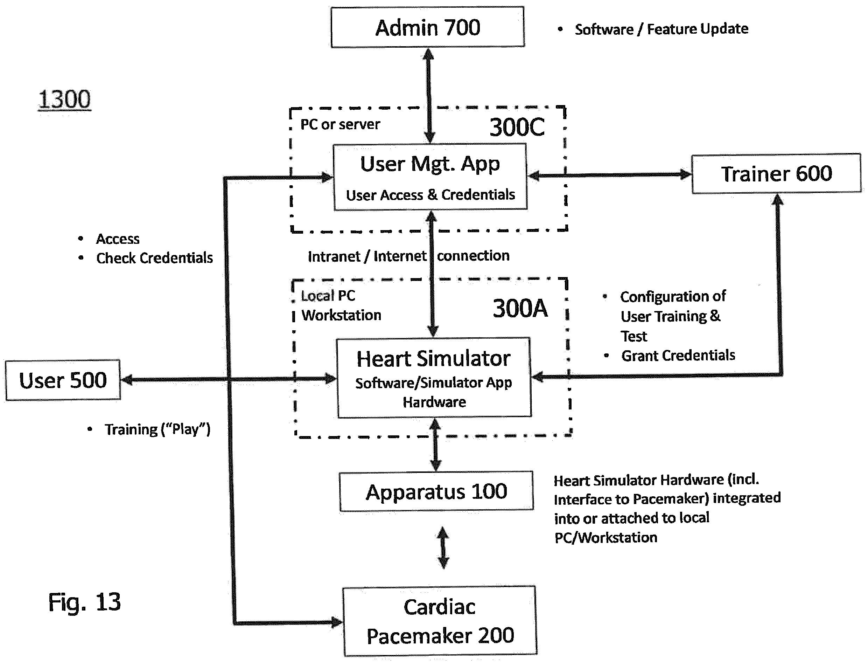

According to the set-up 1300 of Fig. 13, the PC 300A (see Fig. 10) or, alternatively, a workstation hosts a simulator application with the ECG generator 300. The user management application is hosted by a separate PC or server 300C connected to the PC 300A or workstation via the Internet or an intranet.

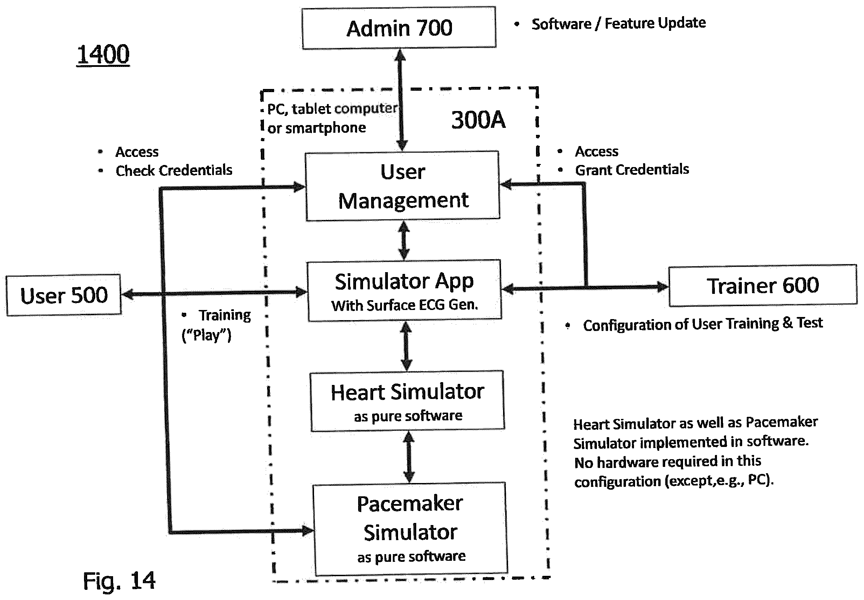

According to the set-up 1400 of Fig. 14, the heart simulator 110 and the pacemaker 200 are configured as software models running on the PC 300A (see Fig. 10), a tablet computer or a smartphone. This means that all the central training services are provided by a single platform.

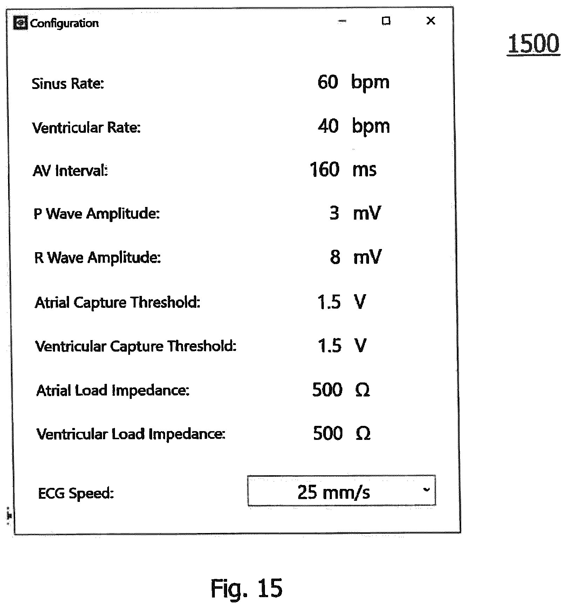

Fig. 15 illustrates a GUI 1500 that serves as an external control interface for controlling, by the trainer 600, the heart simulator 110. The GUI 1500 as external control interface may be coupled by the controller 515 to the heart simulator 100 as described above with reference to Fig. 10.

The GUI 1500 may be generated by the simulator application illustrated in Figs. 12 and 14. Via the GUI 1500, the trainer 600 may vary one, two or more parameters of a set of simulated heart parameters to prompt a reaction by the user 500 to be trained. Such a heart parameter set may include, but is not limited to, sinus rate, ventricular rate, AV interval, P wave amplitude, R wave amplitude, atrial capture threshold, and ventricular capture threshold. Furthermore, electrical properties of the stimulation lead systems may be altered (such as atrial lead impedance and ventricular load impedance, see also the component 510 for load impedance matching in Figs. 5 and 10). Another embodiment (not shown) provides controls to setup "pathologic" scenarios such as, but not limited to, atrial fibrillation, AV- Block, and intentionally adverse settings the user 500 to be trained needs to address via suitable user adjustments. Another embodiment (not shown) may differentiate between "patient" scenarios to practice and play with and "patient" scenarios of evaluation and testing.

The training program or the trainer 600 may configure the heart simulator 110 to generate cardiac events that are indicative of a pathologic behavior of the simulated heart, such as conduction abnormalities or irregular heart beats. As understood herein, conduction abnormalities include all forms of AV blocks, and irregular heart beats include atrial fibrillation.

An exemplary training scenario comprises evaluating the adjustment information. The evaluation may be performed by the training program or by a separate evaluation program hosted externally to the apparatus 100 (e.g., on the PC 300A). Additionally, or in the alternative, the evaluation may be performed by the trainer 600 to whom the adjustment information is presented via a dedicated GUI (e.g., generated by the PC 300A or an another computer operated by the trainer 600).

One goal of the evaluation is to determine (e.g., in real-time or at least in a close temporal relationship to a user adjustment) whether a certain user adjustment performed at the pacemaker, possibly in response to a set of cardiac events indicative of a pathologic behavior of the heart, will therapeutically be effective. A result of this determination may be output as user training information (indicating, e.g., that the training was successful or giving the user feedback about missing adjustments or adjustments that were performed improperly).

As an example, it may be determined if the user 500 has operated a certain control button or control wheel of an external pacemaker correctly, incorrectly or erroneously not at all. As such, the adjustment information may also be indicative of an adjustment that has erroneously not been performed. The adjustment information may generally be indicative of current settings of adjustable pacemaker parameters. In such a case, (lack of) a user adjustment can be determined by a (missing) change of the pacemaker settings over a period of time (e.g., from one parameter setting data set output by the pacemaker to the next such data set).

Various training scenarios may be defined that each comprises a dedicated (lack of) generation of cardiac events to trigger an associated user adjustment. In a first training scenario, the capture threshold is to be determined by the user 500, but the adjustment information indicates that the user 500 has forgotten to adjust the stimulation rate to be higher than the intrinsic rate. In a second training scenario, the adjustment information indicates that the user 500 is further increasing the sensing threshold although the intrinsic activities are no longer detected. In a third training scenario, the adjustment information indicates that the user 500 is further decreasing the sensing threshold although in addition to intrinsic activities, noise or other signal artifacts are already detected. In a fourth training scenario, the pacemaker is to be operated in a demand mode (e.g., detect intrinsic ventricular activities), but the adjustment information indicates that the user 500 has missed to select the AV delay to be longer than the intrinsic AV interval.

In all those training scenarios, an automatic (e.g., program-based) feedback or a feedback from the trainer 600 may be given to the user 500, for example at a GUI (only) presented to the user 500. As such, user training can efficiently be performed in a largely automated way, which increases the user's ability of proper patient treatment.

## Claims

1. A user-adjustable external cardiac pacemaker (200) comprising
a first input interface (210) configured to receive a signal representation of a cardiac event having a pulse amplitude;
a first output interface (212) configured to output one or more stimuli generated by the cardiac pacemaker (200); and
a second output interface (214) configured to output adjustment information indicative of a user adjustment to at least one of
i) an apparatus (100) for controlling operation of the pacemaker (200) in a training mode and
ii) an ECG generator (300) or a dedicated training sub-system.

2. The user-adjustable external cardiac pacemaker (200) of claim 1, wherein
the adjustment information is different from stimulus information pertaining to the one or more stimuli generated by the cardiac pacemaker (200).

3. The user-adjustable external cardiac pacemaker (200) of any of the preceding claims, comprising
a user interface for performing user adjustments controlling operation of the pacemaker (200); and
electric circuitry for transforming the user adjustments into the adjustment information.

4. The user-adjustable external cardiac pacemaker (200) of claim 3, wherein the adjustment information is time-stamped.

5. The user-adjustable external cardiac pacemaker (200) of claim 3, wherein
the adjustment information is indicative of one or more adjustment parameters and wherein each adjustment parameter is associated with a time stamp in the adjustment information.

6. The user-adjustable external cardiac pacemaker (200) of claim 3 or 4, wherein the user interface is a mechanical user interface.

7. The user-adjustable external cardiac pacemaker (200) of any of the preceding claims, wherein
the first output interface (212) is an analog interface.

8. The user-adjustable external cardiac pacemaker (200) of any of the preceding claims, wherein
the first output interface (212) is configured to be coupled to an apparatus (100) for controlling operation of the pacemaker (200) in a training mode.

9. The user-adjustable external cardiac pacemaker (200) of any of the preceding claims, wherein
the second output interface (214) is a digital interface.

10. The user-adjustable external cardiac pacemaker (200) of any of the preceding claims, wherein
the second output interface (214) is configured to be coupled to an apparatus (100) for controlling operation of the pacemaker (200) in a training mode.

11. A method of using the adjustment information output by the user-adjustable
external cardiac pacemaker (200) of any of the preceding claims in a training mode, the method comprising
receiving the adjustment information; and
evaluating the adjustment information with respect to an expected user adjustment.

12. The method of claim 11, comprising
generating user training information indicative of a result of the evaluation.

13. The method of claim 11 or 12, wherein
a heart simulator is configured to execute a training program simulating the cardiac event and wherein the cardiac event is associated with one or more expected user adjustments, and wherein the adjustment information is evaluated to determine deviations between the one or more user adjustments comprised in the adjustment information and the one or more expected user adjustments.

14. The method of claims 12 and 13, wherein
the deviations form the basis of the user training information.

## Patentansprüche

1. Benutzereinstellbarer externer Herzschrittmacher (200), umfassend
eine erste Eingabeschnittstelle (210), die ausgebildet ist, um eine Signaldarstellung eines kardialen Ereignisses mit einer Pulsamplitude zu empfangen;
eine erste Ausgabeschnittstelle (212), die ausgebildet ist, um einen oder mehrere von dem Herzschrittmacher (200) erzeugte Stimuli auszugeben; und
eine zweite Ausgabeschnittstelle (214), die ausgebildet ist, um Einstellungsinformationen auszugeben, die eine Benutzereinstellung an i) einer Vorrichtung (100) zum Steuern des Betriebs des Herzschrittmachers (200) in einem Trainingsmodus und/oder ii) einem EKG-Generator (300) oder einem dedizierten Trainings-Subsystem angeben.

2. Benutzereinstellbarer externer Herzschrittmacher (200) nach Anspruch 1, wobei sich die Einstellungsinformationen von Stimulusinformationen unterscheiden, die sich auf den einen oder die mehreren von dem Herzschrittmacher (200) erzeugten Stimuli beziehen.

3. Benutzereinstellbarer externer Herzschrittmacher (200) nach einem der vorhergehenden Ansprüche, umfassend
eine Benutzerschnittstelle zum Durchführen von Benutzereinstellungen, die den Betrieb des Herzschrittmachers (200) steuern; und
eine elektrische Schaltung zum Umwandeln der Benutzereinstellungen in die Einstellungsinformationen.

4. Benutzereinstellbarer externer Herzschrittmacher (200) nach Anspruch 3, wobei die Einstellungsinformationen mit einem Zeitstempel versehen sind.

5. Benutzereinstellbarer externer Herzschrittmacher (200) nach Anspruch 3, wobei die Einstellungsinformationen einen oder mehrere Einstellungsparameter angeben und wobei jeder Einstellungsparameter einem Zeitstempel in den Einstellungsinformationen zugeordnet ist.

6. Benutzereinstellbarer externer Herzschrittmacher (200) nach Anspruch 3 oder 4, wobei die Benutzerschnittstelle eine mechanische Benutzerschnittstelle ist.

7. Benutzereinstellbarer externer Herzschrittmacher (200) nach einem der vorhergehenden Ansprüche, wobei die erste Ausgabeschnittstelle (212) eine analoge Schnittstelle ist.

8. Benutzereinstellbarer externer Herzschrittmacher (200) nach einem der vorhergehenden Ansprüche, wobei die erste Ausgabeschnittstelle (212) ausgebildet ist, um mit einer Vorrichtung (100) gekoppelt zu werden, um den Betrieb des Herzschrittmachers (200) in einem Trainingsmodus zu steuern.

9. Benutzereinstellbarer externer Herzschrittmacher (200) nach einem der vorhergehenden Ansprüche, wobei die zweite Ausgabeschnittstelle (214) eine digitale Schnittstelle ist.

10. Benutzereinstellbarer externer Herzschrittmacher (200) nach einem der vorhergehenden Ansprüche, wobei die zweite Ausgabeschnittstelle (214) ausgebildet ist, um mit einer Vorrichtung (100) gekoppelt zu werden, um den Betrieb des Herzschrittmachers (200) in einem Trainingsmodus zu steuern.

11. Verfahren zur Verwendung der Einstellungsinformationen, die von dem von dem benutzereinstellbaren externen Herzschrittmacher (200) nach einem der vorhergehenden Ansprüche in einem Trainingsmodus ausgegebenen werden, wobei das Verfahren umfasst:
Empfangen der Einstellungsinformationen; und
Auswerten der Einstellungsinformationen in Bezug auf eine erwartete Benutzereinstellung.

12. Verfahren nach Anspruch 11, umfassend
Erzeugen von Benutzertrainingsinformationen, die ein Ergebnis der Auswertung angeben.

13. Verfahren nach Anspruch 11 oder 12, wobei
ein Herzsimulator ausgebildet ist, um ein Trainingsprogramm auszuführen, das das kardiale Ereignis simuliert, und wobei das kardiale Ereignis einer oder mehreren erwarteten Benutzereinstellungen zugeordnet ist, und wobei die Einstellungsinformationen ausgewertet werden, um Abweichungen zwischen der einen oder den mehreren Benutzereinstellungen, die in den Einstellungsinformationen umfasst sind, und der einen oder den mehreren erwarteten Benutzereinstellungen zu bestimmen.

14. Verfahren nach Ansprüchen 12 und 13, wobei
die Abweichungen die Grundlage für die Benutzertrainingsinformationen bilden.

## Revendications

1. Stimulateur cardiaque externe réglable par l'utilisateur (200) comprenant
une première interface d'entrée (210) configurée pour recevoir un signal représentant un événement cardiaque ayant une hauteur d'impulsions;
une première interface de sortie (212) configurée pour émettre un ou plusieurs stimuli générés par le stimulateur cardiaque (200); et
une deuxième interface de sortie (214) configurée pour délivrer des informations de réglage indiquant un réglage de l'utilisateur au moins de l'un parmi
i) un appareil (100) destiné à commander le fonctionnement du stimulateur cardiaque (200) dans un mode d'entraînement et
ii) un générateur ECG (300) ou un sous-système d'entraînement dédié.

2. Stimulateur cardiaque externe réglable par l'utilisateur (200) selon la revendication 1, dans lequel
les informations de réglage sont différentes des informations relatives au stimulus se rapportant à un ou plusieurs stimuli générés par le stimulateur cardiaque (200).

3. Stimulateur cardiaque externe réglable par l'utilisateur (200) selon l'une quelconque des revendications précédentes, comprenant
une interface utilisateur destinée à effectuer les réglages d'utilisateur commandant le fonctionnement du stimulateur cardiaque (200); et
un circuit électrique destiné à transformer les réglages d'utilisateur en informations de réglage.

4. Stimulateur cardiaque externe réglable par l'utilisateur (200) selon la revendication 3, dans lequel
les informations de réglage sont des estampilles temporelles.

5. Stimulateur cardiaque externe réglable par l'utilisateur (200) selon la revendication 3, dans lequel
les informations de réglage indiquent un ou plusieurs paramètres de réglage et dans lequel chaque paramètre de réglage est associé à une estampille temporelle dans les informations de réglage.

6. Stimulateur cardiaque externe réglable par l'utilisateur (200) selon la revendication 3 ou 4, dans lequel
l'interface utilisateur est une interface mécanique.

7. Stimulateur cardiaque externe réglable par l'utilisateur (200) selon l'une quelconque des revendications précédentes, dans lequel
la première interface de sortie (212) est une interface analogique.

8. Stimulateur cardiaque externe réglable par l'utilisateur (200) selon l'une quelconque des revendications précédentes,
la première interface de sortie (212) est configurée pour être couplée à un appareil (100) permettant de commander le fonctionnement du stimulateur cardiaque (200) dans un mode d'entraînement.

9. Stimulateur cardiaque externe réglable par l'utilisateur (200) selon l'une quelconque des revendications précédentes, dans lequel
la deuxième interface de sortie (214) est une interface numérique.

10. Stimulateur cardiaque externe réglable par l'utilisateur (200) selon l'une quel-
conque des revendications précédentes, dans lequel
la deuxième interface de sortie (214) est configurée pour être couplée à un appareil (100) permettant de commander le fonctionnement du stimulateur cardiaque (200) dans un mode d'entraînement.

11. Procédé d'utilisation des informations de réglage délivrées par le stimulateur cardiaque externe réglable par l'utilisateur (200) selon l'une des revendications précédentes dans un mode d'entraînement, le procédé comprenant
la réception des informations de réglage; et
l'évaluation des informations de réglage par rapport à un réglage attendu de l'utilisateur.

12. Procédé selon la revendication 11, comprenant
la génération d'informations d'entraînement d'utilisateur indiquant un résultat de l'évaluation.

13. Procédé selon la revendication 11 ou 12, dans lequel un simulateur cardiaque est configuré pour exécuter un programme d'entraînement simulant l'événement cardiaque et dans lequel l'événement cardiaque est associé à un ou plusieurs réglages attendus de l'utilisateur, et dans lequel les informations de réglage sont évaluées pour déterminer les écarts entre le ou les réglages de l'utilisateur compris dans les informations de réglage et le ou les réglages attendus de l'utilisateur.

14. Procédé selon la revendication 12 et 13, dans lequel
les écarts constituent la base des informations relatives à l'entraînement d'utilisateur.
